# EUROPEAN PATENT SPECIFICATION

(11) **EP 3 993 859 B1**
(45) Date of publication and mention of the grant of the patent: **14.09.2022**
(21) Application number: 20731880.9
(22) Date of filing: 15.06.2020
(51) Int. Cl.: A61M 15/02

(54) **PLASMA ENHANCED AEROSOL DEVICE**
PLASMAVERSTÄRKTE AEROSOLVORRICHTUNG
DISPOSITIF AÉROSOL À PLASMA AMÉLIORÉ

(30) Priority: 01.07.2019 EP 19183609
(43) Date of publication of application: 11.05.2022
(73) Proprietor: Universiteit Gent, 9000 Gent (BE)
(72) Inventor: DE GEYTER, Nathalie, 8920 Langemark (BE); COOLS, Pieter, 9880 Aalter (BE); NIKIFOROV, Anton, 9000 Gent (BE); MORENT, Rino, 8920 Langemark (BE)
(74) Representative: DenK iP bv
(86) International application number: PCT/EP2020/066530
(87) International publication number: WO 2021/001139

(56) References cited:
- WO-A1-2010/146438
- WO-A2-2012/104332
- US-A1- 2016 106 993
- US-A1- 2016 193 336
- "PLASMA GESUNDHEITSFÜRSORGE, PERSÖNLICHE HYGIENE", INTERNET CITATION, 1 January 2011 (2011-01-01), pages 1-19, XP002719226, Retrieved from the Internet: URL:http://www.mpe.mpg.de/882068/PlasmaMed izin_Broschuere.pdf [retrieved on 2014-01-24]

## Description

### Field of the invention

The invention relates to the field of aerosol devices. More specifically it relates to aerosol devices adapted for user inhalation of a generated aerosol.

### Background of the invention

Cystic fibrosis is a genetic disease that affects the Chlorine transport in the lungs. The cells that produce mucus are affected by this and this leads to abnormally viscous mucus production. This mucus remains in the lungs and is a source of bacterial infections.

Current treatment schemes for (chronic) lung infections involve the use of:
- Antibiotics (oral intake, intravenous intake or aerosol-based via inhalation);
- Dornase alfa (Pulmozyme^{®}), which is an enzymatic "scissor" that helps in the drainage of (infected) mucus;
- Salbutamol (Ventolin), which is a pharmaceutical compound used for opening up the airways, to facilitate breathing;
- Intense kinetherapy for stimulated drainage of the mucus.

Of those four treatment pathways, only the first actively targets bacterial infections and is a pharma- based approach that induces the risk of multidrug resistant (MDR) bacterial development in the patient's lungs.

In view of this risk there is a need an aerosol device for user inhalation of an aerosol stream which provides an additional pathway for treating chronic lung infections.

WO2010/146438A1 discloses a plasma coating device for treating a wound. US2016193336A1 discloses a method for administering nitric oxide in a plasma state to an organism.

Applications of plasma in medicine are also described in a brochure "PLASMA GESUNDHEITS- FÜRSORGE" by Max-Planck-Institute (XP002719226).

### Summary of the invention

It is an object of embodiments of the present invention to provide a good aerosol device for user inhalation of an aerosol stream.

The above objective is accomplished by a method and device according to the present invention.

In a first aspect embodiments of the present invention relate to an aerosol device for user inhalation of an aerosol stream. The device comprises:
- an aerosol inhaler adapted for guiding the aerosol stream comprising a plurality of microdroplets,
- a non-thermal plasma jet generator adapted for generating a plasma plume of non-thermal plasma. The plasma jet generator comprises an outlet for exhausting the plasma plume in the aerosol stream.

It is an advantage of embodiments of the present invention that by generating the plasma plume in the aerosol stream, the microdroplets in this aerosol stream will be activated by the plasma jet. Thus, an aerosol stream is created with radicals inside.

It is an advantage of embodiments of the present invention that they allow to activate the aerosols using non-thermal plasma. Thereby active components of the non-thermal plasma are transferred to the aerosols.

This is particularly advantageous because aerosols can be inhaled by the patient and, as they are activated, they will break down the bacteria in the lungs. Hence it is possible to break down the bacteria on a physical chemical way. This can be combined with a pharmaceutical treatment using antibiotics or can be used as an alternative to antibiotics. Less high doses of antibiotics will be required because of the combination of the aerosols which are activated with the non-thermal plasma. Hence, the formation of resistant bacteria will be strongly reduced.

In embodiments of the present invention the outlet of the plasma jet generator is oriented such that, in operation, the plasma plume is oriented substantially in the direction of the aerosol stream.

It is an advantage of embodiments of the present invention that a more reproducible mixing of the aerosol with the plasma jet is obtained than in case the plasma plume would be oriented crosswise on the aerosol stream. In the latter case the plasma plume will cause turbulence in the aerosol stream. In the former case interaction between the plasma and the aerosol stream happens through diffusion.

To efficiently the plasma jet and the aerosol stream, the skilled person would be inclined to insert the plasma jet orthogonal with the aerosol stream. However, the inventors have found out that better results, especially in relation to spikes in the concentration of harmful components, can be obtained by not disturbing the flow. Spikes in the concentration of harmful components can be strongly reduced by orienting the plasma plume substantially in the direction of the aerosol stream. ^{∗∗∗})

In embodiments of the present invention the non-thermal plasma jet generator comprises a plurality of outlets.

In embodiments of the present invention the plasma jet generator may for example comprise a ring with several holes such that plasma plumes can exit the ring through the plurality of outlets. The outlets may thereby be oriented such that each plasma plume is oriented substantially parallel with the aerosol stream.

In embodiments of the present invention the aerosol device may comprise one outlet in the center of the aerosol stream.

By putting an outlet in the center of the aerosol stream a higher number of aerosols can be affected by the plasma plume, which is substantially parallel with the aerosol stream, than when the outlet is on the side of the aerosol stream.

In embodiments of the present invention the non-thermal plasma jet generator comprises a hollow tube adapted for guiding the plasma towards the outlet and the hollow tube is adapted for changing the orientation of the plasma plume.

In embodiments of the present invention the hollow tube may have an L-shape. This permits to integrate the plasma jet generator in a commercially available aerosol device, while ensuring that the plasma plume is oriented substantially in the direction of the aerosol stream.

In embodiments of the present invention the non-thermal plasma jet generator comprises a first electrode and a second electrode isolated from the first electrode. The electrodes are configured such that a gas can be introduced between the electrodes.

The first electrode may be a needle electrode and the second electrode a cylindrical electrode enclosing the needle electrode, with a dielectric capillary between both electrodes and with the needle electrode in the dielectric capillary.

In embodiments of the present invention the plasma jet generator is adapted for introducing the gas in the hollow dielectric capillary.

In embodiments of the present invention the dielectric capillary extends beyond the needle electrode.

In embodiments of the present invention this may be achieved by making a longer dielectric capillary than the length of the needle electrode. Alternatively this may be obtained by adding a hollow tube at the end of the dielectric capillary. This extension may be deformable or may have a pre-defined shape.

In embodiments of the present invention the capillary may be a straight capillary.

In embodiments of the present invention the capillary may be a bended capillary.

In embodiments of the present invention the aerosol device comprises a voltage generator adapted for applying a voltage between the first and second electrode.

In embodiments of the present invention the aerosol device comprises an aerosol generator adapted for generating the aerosol stream in the aerosol inhaler.

In embodiments of the present invention the aerosol device comprising at least one controller adapted for monitoring and/or controlling the aerosol device.

In embodiments of the present invention the at least one controller may be adapted for controlling a gas flow generator and for controlling a plasma discharge in this gas flow.

In embodiments of the present invention the at least one controller may be adapted for monitoring whether or not an aerosol stream is flowing and for activating the plasma discharge only if the aerosol stream is flowing.

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiment(s) described hereinafter.

### Brief description of the drawings

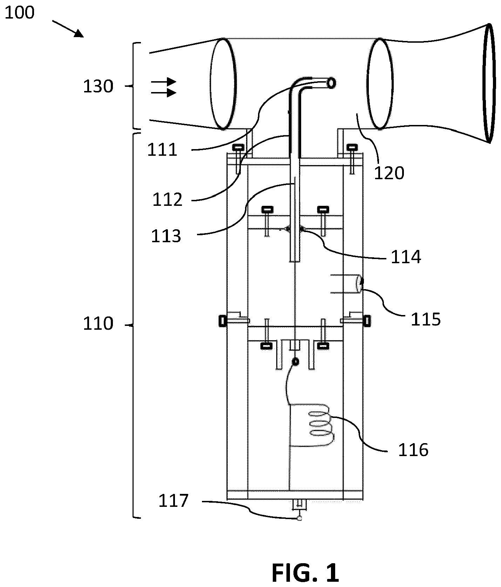

FIG. 1 shows a schematic drawing of an aerosol device in accordance with embodiments of the present invention.

Any reference signs in the claims shall not be construed as limiting the scope. In the different drawings, the same reference signs refer to the same or analogous elements.

### Detailed description of illustrative embodiments

The present invention will be described with respect to particular embodiments and with reference to a drawing but the invention is not limited thereto but only by the claims. The drawing described is only schematic and is non-limiting. In the drawing, the size of some of the elements may be exaggerated and not drawn on scale for illustrative purposes. The dimensions and the relative dimensions do not correspond to actual reductions to practice of the invention.

The terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

In the description provided herein, numerous specific details are set forth. However, it is understood that embodiments of the invention may be practiced without these specific details. In other instances, well-known methods, structures and techniques have not been shown in detail in order not to obscure an understanding of this description.

Where in embodiments of the present invention reference is made to an aerosol stream reference is made to a stream of an aqueous suspension of liquid microdroplets, in air or another gas mixture. In embodiments of the present invention the size of the microdroplets may for example vary between 1 and 10 µm, for example between 2and 5 µm. The standard deviation may for example be 2.5 µm.

In a first aspect embodiments of the present invention relate to an aerosol device 100 for user inhalation of an aerosol stream. The device comprises an aerosol inhaler 130 adapted for guiding the aerosol stream. This aerosol stream comprises a plurality of microdroplets. The aerosol device 100, moreover, comprises a non-thermal plasma jet generator 110 for generating a plasma plume. The plasma jet generator comprises an outlet 111 for exhausting the plasma plume in the aerosol stream.

An example of such an aerosol device, in accordance with embodiments of the present invention, is schematically represented in FIG. 1. The top horizontal part shows the aerosol inhaler 130 which is adapted for guiding an aerosol stream. In this exemplary embodiment the aerosol stream enters the inhaler 130 at a first side (the left side in FIG. 1) of the inhaler and leaves the inhaler at a second side (the right side in FIG. 1) of the inhaler. A mouth piece may be connected to the second side. Through this mouth piece a patient can inhale the activated aerosol stream.

The middle vertical part in this figure is the non-thermal plasma jet generator 110. The outlet of this plasma jet generator is positioned such that, in operation, a plasma plume is generated in the aerosol stream.

Thus, an activated aerosol stream is created. When inhaling this activated aerosol stream bacterial infections in the patients lungs are physio-chemically attacked, thus avoiding the possibility of MDR bacteria development. More so, it would be an approach to get a hold on such infections.

An aerosol device according to embodiments of the present invention, therefore, allows to suppress infections in the lungs. For example chronic infections in the lungs of CF patients can be suppressed, avoiding intense antibiotic schemes. An aerosol device in accordance with embodiments of the present invention may be used in combination with other patient treatment schemes.

When a user inhales an aerosol stream from an aerosol device in accordance with embodiments of the present invention, the time frame between activation of the liquid to bacterial delivery is smaller than a few seconds. It may even be smaller than a second.

In embodiments of the present invention the plasma jet generator 110 is adapted for generating non-thermal plasma. The plasma is obtained by adding energy to the gas phase. In plasma jet generators according to the present invention this is not done thermally, but by applying an alternating electric field. By adding sufficient energy to the gas it can at least be partially ionized (typically less than 1%). Thus, a reactive substance which comprises charged particles, free radicals, and photons is obtained. A mixture may be obtained which in total is electrically neutral. Besides the electrons and radicals, also electromagnetic radiation may be induced. In embodiments of the present invention the plasma is at atmospheric pressure. As no thermal energy is added to the gas the plasma temperature is not significantly increased compared to plasma generators where the plasma is generated by adding thermal energy. Cold plasma, also referred to as non-thermal plasma, may have a temperature below 60°C, or even below 40 °C, for example around 30°C. This plasma leaves the plasma generator through a capillary or through a hollow tube 112 having the same dimensions as the capillary, as a plasma plume.

In embodiments of the present invention the plasma jet generator is oriented such that, in operation, the plasma plume is oriented substantially in the direction of the aerosol stream.

By introducing the plasma plume in the aerosol stream, substantially in the direction of the aerosol stream, a stable interaction between the plasma and the aerosol is created, finally leading to a plasma activated aerosol, which, upon inhalation, is capable of physically attacking/destroying bacterial colonies/biofilms residing in the patient's lungs, while preserving the surrounding lung tissue.

As the outlet is oriented such that the plasma plume is oriented substantially in the direction of the aerosol stream, plasma extinguishing because of the aerosols can be avoided. Moreover, condensation of the aerosols in the outlet of the plasma jet can also be avoided by orienting the outlet such that the plasma plume is oriented substantially in the direction of the aerosol stream plasma. Thus, the fall-out of the plasma all together can be avoided and the reproducibility can be increased compared to a plasma jet generator of which the plasma plume is not oriented in substantially the same direction as the aerosol stream.

By orienting the plasma plume substantially in the direction of the aerosol stream, turbulence in the stream caused by this plasma plume can be reduced compared to embodiments where the plasma plume is oriented orthogonal to the aerosol stream. Spikes in the concentration of harmful components can be reduced by reducing the turbulence in the aerosol stream. For example, the spikes in the ozone concentration should remain below a predefined level to avoid damage of the patients lungs when inhaling this mixture. It is important that the concentrations of various species in the aerosol stream can be maintained within preferred limits. The concentrations of some species must for example be maintained below allowed concentration levels. For example the ozone, hydrogen peroxide, nitrate concentrations must remain within specific limitations.

In embodiments of the present invention the plasma plume may for example form an angle of smaller that 45°, or even smaller than 30°, or even smaller than 15°, or even smaller than 5° such that spikes in the concentration of harmful components are reduced.

A plasma jet generator according to embodiments of the present invention may comprise a plasma source, a capillary part, and a gas inlet.

FIG. 1 shows a schematic drawing of an exemplary aerosol device 100 in accordance with embodiments of the present invention. The aerosol device 100 comprises an aerosol inhaler 130 and a plasma jet generator 110.

The plasma jet generator 110 comprises a plasma source 113, 114, a capillary part 112 an outlet 111 of the capillary part 112, and a gas inlet 115. The plasma jet generator, moreover, comprises a connector 117 for connecting a voltage generator such that a voltage can be applied over the electrodes. In this example the coil 116 is a high voltage transformer to avoid high voltage cables outside of the device for safety.

The plasma jet generator is integrated in the aerosol inhaler 130 such that a plasma plume can be exhausted in the aerosol stream.

In embodiments of the present invention the non-thermal plasma jet generator 130 comprises a dielectric barrier discharge configuration which comprises a first electrode 113 and a second electrode 114 with dielectric material between the electrodes. The dielectric discharge configuration may be assembled such that the dielectric material is covering one of the electrodes. The electrodes are made of conductive material. They may for example comprise a metal.

The plasma jet generator is configured such that a gas can be introduced between the electrodes.

The plasma jet generator may comprise a voltage generator for applying a high alternating voltage of some hundreds of volts up to tens of kilovolts to the conducting electrodes which are isolated from each other. A high voltage is applied such that a discharge is generated in the gas between the electrodes. This voltage generator may be adapted for generating a unipolar, a bipolar or a pulsed voltage with frequency from some Hz to MHz range. A voltage may for example be applied to the first electrode while the second electrode is grounded, or vice versa.

In embodiments of the present invention the dielectric barrier discharge configuration may comprise a capillary section, wherein the first electrode is a needle electrode 113 and the second electrode is a cylindrical electrode 114 enclosing the needle electrode with a capillary made of dielectric material in between both electrodes (i.e. the needle electrode is in the hollow capillary and the hollow capillary is inside the cylindrical electrode). In embodiments of the present invention the capillary is made of dielectric material. It may for example be made of glass, quartz, and medical grade polymers.

The needle electrode may for example have a diameter in the range between a few micrometer to 3 mm. The internal diameter of the dielectric capillary may for example be in the range between 0.1 and 5 mm, with the condition that it should be bigger than the diameter of the needle electrode. The external diameter of the dielectric capillary may for example be a diameter in the range between 0.15 to 7 mm, with the condition that it should be bigger than the internal diameter of the dielectric capillary. The cylindrical electrode 114 is placed around the dielectric capillary 112.

The surface area of the outlet 111 should be small enough in order to have a plasma plume exiting the capillary wherein the plume is sufficiently long to interact with the plasma. The plasma jet generator may for example be adapted for generating a plasma plume with a length more than 1 cm, or even more than 2 cm, or even more than 3 cm. The length of the plasma plume may for example be between 1 and 3 cm.

The dielectric capillary 112 may extend further than the needle electrode 113, thus forming a hollow tube which extends from the end of the needle electrode 113. Alternatively a separate hollow tube 112 may form an extension of the dielectric capillary. The internal diameter of the separate hollow tube may be the same as the internal diameter of the capillary. The separate hollow tube is a tube of dielectric material. It may for example be a plastic hollow tube. The hollow tube which extends from the end of the needle electrode may for example have a length up to 20 cm, or even up to 25 cm, or even up to 30 cm.

In the exemplary embodiment of FIG. 1 the dielectric capillary extends further than the needle electrode 113 and forms a hollow tube 112 which extends from the end of the needle electrode. In this example, this hollow tube is L-shaped. The outlet 111 is oriented such that, in operation, the plasma plume is oriented substantially in the direction of the aerosol stream. This is achieved by the L-shape of the hollow tube of the plasma jet generator. This is particularly advantageous because it guarantees the continuous and reproducible propagation of the plasma into the aerosol without the risk of plasma extinguishing or aerosol condensate traveling inside the capillary, thus altering the composition prior to leaving the capillary.

In general, the hollow tube, may be a flexible tube. By bending this tube the orientation of the plasma plume can be changed.

Although, the extending hollow tube extends the length before the plasma plume can exit the plasma jet generator via the outlet 111, and thus increases the required power for generating the plasma plume, it has the advantage that the plasma plume can be oriented in a preferential direction.

In embodiments of the present invention the plasma jet generator and the aerosol inhaler are configured such that, in operation, the plasma plume is oriented substantially in the direction of the aerosol stream and such that the plasma jet generator comprises a straight capillary. This can for example be achieved by codesigning the aerosol inhaler and the plasma jet generator, in stead of using a prior art aerosol inhaler. The advantage of such a straight capillary is that less energy is required for creating the plasma plume.

In embodiments of the present invention the gas for generating the plasma may for example be Helium or another inert gas or a combination. Besides inert gases other additives may be present in the gas of the cold plasma jet generator.

In embodiments of the present invention the plasma jet generator 110 may comprise an automated gas valve 115 connected on 1 side to the gas capillary 112 and on the other side to a gas canister. The gas canister preferably comprises He, Ar or Ne, or mixtures of those with oxygen, NOₓ, air or other gases with up to 5 vol. % admixing. The gas valve 115 can for example comprise a standard M6 connection to an Air-Liquide He canister of size B10-B50. The invention is, however, not limited to this type of canister. Also the connection system may have different embodiments.

In embodiments of the present invention the plasma jet generator is a non-thermal plasma pen.

In the exemplary embodiment illustrated in FIG. 1 the integration between the aerosol inhaler 130 and the plasma jet generator 110 is achieved by the adapter 120, which is a T-shaped tube configuration comprising a first tube and a second tube. The incoming aerosol stream enters the first tube on one side and exits the first tube on an other side. The second tube is connected with the first tube, between the first and the second side of the first tube, such that the capillary part 112 of the plasma jet generator can be inserted in the first tube via the second tube. In the exemplary embodiment of FIG. 1 the T-shaped extension of the aerosol exit fits over the L-shaped quartz capillary 112, resulting in the capillary being positioned in the middle of the aerosol stream with the plasma exhaust entering the stream in parallel, flowing in the same direction as the aerosol. The extension can be connected to a mouth piece through which the patient can inhale the activated aerosol stream. The T-shaped extension may for example be fabricated out of glass. The invention is, however, not limited thereto. Other types of materials, which are typically used for aerosol inhalers, may be used to fabricate the T-shaped extension. The T-shaped extension is designed to integrate the plasma jet generator in a prior art inhaler. Also, the T-shape of the extension is not strictly required and the extension may be more ergonomically shaped.

In embodiments of the present invention the aerosol inhaler and the plasma jet generator may be designed such that, during operation, the plasma plume is injected in the aerosol stream. Thereby the presence of a T-shaped extension is not necessarily required. The aerosol device comprising the plasma jet generator and the aerosol inhaler may be designed as a handheld device that is ergo-friendly.

In embodiments of the present invention different types of plasma jet generators or plasma source may be used. Depending on the plasma source a different composition of the active species in the aerosol stream may be obtained. This is translated in the composition of the plasma exposed aerosol.

In embodiments of the present invention different active species may be present. Possible active species with preferred ranges of these active species within the aerosol are specified in the list below:
- Ozone between: 0.1-0.3 ppm. Exceeding those concentrations can be harmful to lungs of the host;
- Hydrogen peroxide: 1-20 ppm;
- Nitric acid concentration: 0.1-4 ppm;
- Chlorine dioxide: 0-0.3 ppm;
- NaCl: 1-6%;
- pH: a reduced pH is not advised. Addition of sodium bicarbonate might be needed.

In embodiments of the present invention the plasma jet generator is tuned in terms of power of the plasma plume and in terms of gas flow in order to reach the predefined limitations in the active species.

As discussed before, some of the generated active species transferred into the aerosol could be potentially harmful to lung tissue. A better control of the concentrations can be achieved by orienting the outlet of the plasma jet generator such that the plasma plume is oriented substantially in the same direction as the aerosol stream. The concentrations can, moreover, be better controlled by automating the plasma treatment of the aerosol. The aerosol device may therefore comprise an automated gas valve. The aerosol device may, furthermore, comprise one or more controllers that communicate with the aerosol device. These one or more controllers may for example be adapted for controlling the automated gas valve and/or for controlling the voltage over the electrodes of the plasma jet generator.

In embodiments of the present invention the aerosol device may be adapted to patient-specific parameters such as lung volume, O2 saturation etc.

The aerosol device may comprise a safety module adapted for preventing that a plasma plume is generated when the aerosol stream is not flowing, to avoid that ionized He and air are entering the lungs. Such a safety module may be implemented by the one or more controllers that communicate with the aerosol device (e.g. monitoring/controlling the operational status of the inhaler, monitoring/controlling the operational status of the plasma jet generator, measuring the aerosol stream).

In embodiments of the present invention the one or more controllers are adapted for controlling a gas flow (e.g. He flow) of the plasma jet generator and for controlling a plasma discharge in this gas flow.

In embodiments of the present invention a computer program is implemented on the one or more controllers to control the plasma and/or to control the aerosol stream. Thus a pre-defined plasma activated aerosol stream can be obtained. In embodiments the control of the aerosol inhaler and the control of the plasma jet generator may be achieved by separate controllers and/or by separate processes running on the controllers. These processes controlling the aerosol stream and the plasma plume are preferably linked to guarantee no unintentional use of the plasma jet generator without the flow of an aerosol.

In embodiments of the present invention the aerosol inhaler may be a commercially available aerosol inhaler.

In embodiments of the present invention the aerosol device comprises an aerosol generator adapted for generating the aerosol stream in the aerosol inhaler. Examples of such aerosol generators are the e-flow (PARI GMBH), the Aeroneb (Hamilton) or the I-Neb (Respironics/Philips). Typical aerosol streams consist of aqueous solutions enriched with salts (0.9-6% saline) possibly in combination with a pharma compound such as antibiotics, pulmozyme or salbutamol.

## Claims

1. An aerosol device (100) for user inhalation of an aerosol stream, the device comprising:
- an aerosol inhaler (130) adapted for guiding the aerosol stream comprising a plurality of microdroplets,
**characterised in that** the device comprises:
- a non-thermal plasma jet generator (110) adapted for generating a plasma plume of non-thermal plasma, the plasma jet generator (110) comprising an outlet (111) for exhausting the plasma plume in the aerosol stream.

2. An aerosol device (100) according to claim 1, wherein the outlet (111) of the plasma jet generator (110) is oriented such that, in operation, the plasma plume is oriented substantially in the direction of the aerosol stream.

3. An aerosol device (100) according to any of the previous claims wherein the non-thermal plasma jet generator (110) comprises a plurality of outlets.

4. An aerosol device (100) according to any of the previous claims comprising one outlet in the center of the aerosol stream.

5. An aerosol device (100) according to any of the previous claims, wherein the non-thermal plasma jet generator (110) comprises a hollow tube adapted for guiding the plasma towards the outlet and wherein the hollow tube is adapted for changing the orientation of the plasma plume.

6. An aerosol device (100) according to any of the previous claims, wherein the non-thermal plasma jet generator (110) comprises a first electrode (113) and a second electrode (114) isolated from the first electrode (113), which are configured such that a gas can be introduced between the electrodes (113, 114).

7. An aerosol device according to claim 6, wherein the first electrode 113 is a needle electrode and wherein the second electrode 114 is a cylindrical electrode enclosing the needle electrode, with a dielectric capillary between both electrodes and with the needle electrode in the dielectric capillary.

8. An aerosol device according to claim 7, wherein the dielectric capillary extends beyond the needle electrode.

9. An aerosol device according to any of the claims 7 or 8, wherein the capillary is a straight capillary.

10. An aerosol device according to any of the claim 7 or 8, wherein the capillary is a bended capillary.

11. An aerosol device (100) according to any of the claims 6 to 10, the aerosol device (100) comprising a voltage generator adapted for applying a voltage between the first (113) and second electrode (114).

12. An aerosol device (100) according to any of the previous claims, the aerosol device comprising an aerosol generator adapted for generating the aerosol stream in the aerosol inhaler.

13. An aerosol device (100) according to any of the previous claims, the aerosol device comprising at least one controller adapted for monitoring and/or controlling the aerosol device.

14. An aerosol device (100) according to claim 13 wherein the at least one controller is adapted for controlling a gas flow generator and for controlling a plasma discharge in this gas flow.

15. An aerosol device (100) according to claim 14 wherein the at least one controller is adapted for monitoring whether or not an aerosol stream is flowing and for activating the plasma discharge only if the aerosol stream is flowing.

## Patentansprüche

1. Eine Aerosolvorrichtung (100) zur Installation durch einen Benutzer eines Aerosolstroms, wobei die Vorrichtung Folgendes umfasst:
- einen Aerosolinhalator (130), der zum Führen des Aerosolstroms, der eine Vielzahl von Mikrotröpfchen umfasst, angepasst ist,
**dadurch gekennzeichnet, dass** die Vorrichtung Folgendes umfasst:
- einen nicht thermischen Plasmastrahlgenerator (110), der dazu angepasst ist, eine Plasmafahne aus nicht thermischem Plasma zu erzeugen, wobei der Plasmastrahlgenerator (110) einen Auslass (111) zum Ableiten der Plasmafahne in den Aerosolstrom umfasst.

2. Eine Aerosolvorrichtung (100) nach Anspruch 1, wobei der Auslass (111) des Plasmastrahlgenerators (110) derart ausgerichtet ist, dass die Plasmafahne beim Betrieb im Wesentlichen in der Richtung des Aerosolstroms ausgerichtet ist.

3. Eine Aerosolvorrichtung (100) nach einem der vorstehenden Ansprüche, wobei der nicht thermische Plasmastrahlgenerator (110) eine Vielzahl von Auslässen umfasst.

4. Eine Aerosolvorrichtung (100) nach einem der vorstehenden Ansprüche, die einen Auslass in der Mitte des Aerosolstroms umfasst.

5. Eine Aerosolvorrichtung (100) nach einem der vorstehenden Ansprüche, wobei der nicht thermische Plasmastrahlgenerator (110) eine Hohlröhre umfasst, die dazu angepasst ist, das Plasma in Richtung des Auslasses zu führen, und wobei die Hohlröhre dazu angepasst ist, die Ausrichtung der Plasmafahne zu ändern.

6. Eine Aerosolvorrichtung (100) nach einem der vorstehenden Ansprüche, wobei der nicht thermische Plasmastrahlgenerator (110) eine erste Elektrode (113) und eine zweite Elektrode (114), die von der ersten Elektrode (113) isoliert ist, umfasst, die derart konfiguriert sind, dass ein Gas zwischen den Elektroden (113, 114) eingeführt werden kann.

7. Eine Aerosolvorrichtung nach Anspruch 6, wobei die erste Elektrode 113 eine Nadelelektrode ist, und wobei die zweite Elektrode 114 eine zylindrische Elektrode ist, die die Nadelelektrode umschließt, mit einer dielektrischen Kapillare zwischen beiden Elektroden und mit der Nadelelektrode in der dielektrischen Kapillare.

8. Eine Aerosolvorrichtung nach Anspruch 7, wobei sich die dielektrische Kapillare über die Nadelelektrode hinaus erstreckt.

9. Eine Aerosolvorrichtung nach einem der Ansprüche 7 oder 8, wobei die Kapillare eine gerade Kapillare ist.

10. Eine Aerosolvorrichtung nach einem der Ansprüche 7 oder 8, wobei die Kapillare eine gebogene Kapillare ist.

11. Eine Aerosolvorrichtung (100) nach einem der Ansprüche 6 bis 10, wobei die Aerosolvorrichtung (100) einen Spannungsgenerator umfasst, der dazu angepasst ist, eine Spannung zwischen der ersten (113) und der zweiten Elektrode (114) anzulegen.

12. Eine Aerosolvorrichtung (100) nach einem der vorstehenden Ansprüche, wobei die Aerosolvorrichtung einen Aerosolgenerator umfasst, der zum Erzeugen des Aerosolstroms in dem Aerosolinhalator angepasst ist.

13. Eine Aerosolvorrichtung (100) nach einem der vorstehenden Ansprüche, wobei die Aerosolvorrichtung mindestens einen Controller umfasst, der zum Überwachen und/oder Steuern der Aerosolvorrichtung angepasst ist.

14. Eine Aerosolvorrichtung (100) nach Anspruch 13, wobei der mindestens eine Controller zum Steuern eines Gasflussgenerators und zum Steuern einer Plasmaentladung in diesem Gasfluss angepasst ist.

15. Eine Aerosolvorrichtung (100) nach Anspruch 14, wobei der mindestens eine Controller zum Überwachen angepasst ist, ob ein Aerosolstrom fließt oder nicht, und zum Aktivieren der Plasmaentladung nur, falls der Aerosolstrom fließt.

## Revendications

1. Un dispositif aérosol (100) pour l'inhalation par un utilisateur d'un flux d'aérosol, le dispositif comprenant :
- un inhalateur aérosol (130) conçu pour guider le flux d'aérosol comprenant une pluralité de microgouttelettes,
**caractérisé en ce que** le dispositif comprend :
- un générateur de jet de plasma non thermique (110) conçu pour générer un nuage de plasma de plasma non thermique, le générateur de jet de plasma (110) comprenant une sortie (111) destinée à évacuer le nuage de plasma dans le flux d'aérosol.

2. Un dispositif aérosol (100) selon la revendication 1, dans lequel la sortie (111) du générateur de jet de plasma (110) est orientée de sorte que, en fonctionnement, le nuage de plasma soit orienté sensiblement dans la direction du flux d'aérosol.

3. Un dispositif aérosol (100) selon l'une quelconque des revendications précédentes dans lequel le générateur de jet de plasma non thermique (110) comprend une pluralité de sorties.

4. Un dispositif aérosol (100) selon l'une quelconque des revendications précédentes comprenant une sortie au centre du flux d'aérosol.

5. Un dispositif aérosol (100) selon l'une quelconque des revendications précédentes, dans lequel le générateur de jet de plasma non thermique (110) comprend un tube creux conçu pour guider le plasma vers la sortie et dans lequel le tube creux est conçu pour modifier l'orientation du nuage de plasma.

6. Un dispositif aérosol (100) selon l'une quelconque des revendications précédentes, dans lequel le générateur de jet de plasma non thermique (110) comprend une première électrode (113) et une seconde électrode (114) isolée de la première électrode (113), qui sont configurées de sorte qu'un gaz puisse être introduit entre les électrodes (113, 114).

7. Un dispositif aérosol selon la revendication 6, dans lequel la première électrode (113) est une électrode aiguille et dans lequel la second électrode (114) est une électrode cylindrique entourant l'électrode aiguille, un capillaire diélectrique étant compris entre les deux électrodes et l'électrode aiguille étant présente dans le capillaire diélectrique.

8. Un dispositif aérosol selon la revendication 7, dans lequel le capillaire diélectrique s'étend au-delà de l'électrode aiguille.

9. Un dispositif aérosol selon l'une quelconque des revendications 7 ou 8, dans lequel le capillaire est un capillaire droit.

10. Un dispositif aérosol selon l'une quelconque des revendications 7 ou 8, dans lequel le capillaire est un capillaire recourbé.

11. Un dispositif aérosol (100) selon l'une quelconque des revendications 6 à 10, le dispositif aérosol (100) comprenant un générateur de tension conçu pour appliquer une tension entre la première électrode (113) et la seconde électrode (114).

12. Un dispositif aérosol (100) selon l'une quelconque des revendications précédentes, le dispositif aérosol comprenant un générateur d'aérosol conçu pour générer le flux d'aérosol dans l'inhalateur aérosol.

13. Un dispositif aérosol (100) selon l'une quelconque des revendications précédentes, le dispositif aérosol comprenant au moins un dispositif de commande conçu pour surveiller et/ou commander le dispositif aérosol.

14. Un dispositif aérosol (100) selon la revendication 13 dans lequel le au moins un dispositif de commande est conçu pour commander un générateur de flux gazeux et pour commander une décharge de plasma dans ce flux gazeux.

15. Un dispositif aérosol (100) selon la revendication 14 dans lequel le au moins un dispositif de commande est conçu pour surveiller si un flux d'aérosol circule ou non ou pour n'activer la décharge de plasma que si le flux de plasma circule.
